# EUROPÄISCHE PATENTSCHRIFT

(11) **EP 1 663 080 B1**
(45) Veröffentlichungstag und Bekanntmachung des Hinweises auf die Patenterteilung: **23.04.2008**
(21) Anmeldenummer: 03818891.8
(22) Anmeldetag: 27.09.2003
(51) Int. Cl.: A61F 2/46, A61B 19/00, A61B 5/103, A61B 5/107, G01S 5/18

(54) **Verfahren und Vorrichtung zur Bestimmung der Beweglichkeit einer Hüftgelenkprothese**
Method and device for determination of the mobility of a hip joint prosthesis
Procédé et dispositif pour déterminer la mobilité d'une prothèse de la hanche

(43) Veröffentlichungstag der Anmeldung: 07.06.2006
(73) Patentinhaber: AESCULAP AG & Co. KG, 78532 Tuttlingen (DE)
(72) Erfinder: LEITNER, François, F-38410 Uriage (FR); MOLLARD, Benoit, 38130 Echirolles (FR)
(74) Vertreter: Boehme, Ulrich
(86) Internationale Anmeldenummer: PCT/EP2003/010779
(87) Internationale Veröffentlichungsnummer: WO 2005/039456

(56) Entgegenhaltungen:
- WO-A-00/32093
- WO-A-02/071987
- WO-A-02/080824
- DE-A- 19 709 960
- DE-U- 20 016 635
- DE-U- 20 304 153
- DE-U- 20 315 005
- FR-A- 2 770 128
- US-A1- 2003 153 829
- US-B1- 6 205 411

## Beschreibung

Die Erfindung betrifft ein Verfahren zur Bestimmung der Beweglichkeit einer Hüftgelenkprothese mit einer in den Hüftknochen einsetzbaren Lagerschale und einem in den Femur einsetzbaren Prothesenschaft, der mit einer kugeligen Gelenkfläche in der Lagerschale verschwenkbar gelagert ist.

Außerdem betrifft die Erfindung eine Vorrichtung zur Durchführung dieses Verfahrens.

WO-A-02/080824 beschreibt ein Verfahren und eine Vorrichtung zum Anbringen einer Hüftgelenkprothese an einem Körper, mit welchen die Prothese möglichst exakt positioniert werden kann.

Beim Einsetzen von Hüftgelenkprothesen wird üblicherweise in den Hüftknochen eine Lagerschale eingesetzt und in den Femur ein Prothesenschaft, dieser Prothesenschaft greift mit einer kugeligen Gelenkfläche in die ebenfalls kugelig ausgebildete Lagerschale ein, so daß die Mittelpunkte der Lagerschale und der kugeligen Gelenkfiläche des Prothesenschaftes zusammenfallen. Der Femur kann aufgrund dieser Lagerung in allen Richtungen gegenüber dem Hüftknochen verschwenkt werden, diese Verschwenkbewegung wird einmal begrenzt durch das umgebende Weichgewebe und zum anderen kann diese Schwenkbewegung dadurch begrenzt werden, daß die knöchernen Strukturen von Hüftgelenk und Femur aneinander oder an Teilen der Prothese anschlagen. Diese Beschränkung der Schwenkbewegung kann unerwünscht sein, und daher ist es notwendig, bei der Operation festzustellen, ob nach dem Einsetzen der Hüftgelenkprothese die erwünschte Beweglichkeit des Femurs gegenüber dem Hüftknochen erreicht wird.

Der Erfindung liegt demgemäß die Aufgabe zugrunde, ein Verfahren zur Bestimmung der Beweglichkeit einer Hüftgelenkprothese zu schaffen, welches es dem Operateur ermöglicht, den zur Verfügung stehenden Schwenkbereich zu bestimmen.

Diese Aufgabe wird bei einem Verfahren der eingangs beschriebenen Art erfindungsgemäß dadurch gelöst, daß man den Femur mit dem Prothesenschaft in verschiedenen Richtungen so weit wie möglich verschwenkt, daß man feststellt, ob sich dabei der Mittelpunkt der Lagerschale und der Mittelpunkt der kugeligen Gelenkfläche voneinander entfernen, und daß man dadurch für jede Schwenkrichtung den maximalen Schwenkwinkel bestimmt, bei dem die Mittelpunkte beginnen, sich voneinander zu entfernen.

Wenn die Schwenkbewegung des Femurs durch einen der genannten Anschläge begrenzt wird, also durch direkten Kontakt knöcherner Strukturen, durch Kontakt von Prothesenteilen mit knöchernen Strukturen oder durch Kontakt von Prothesenteilen untereinander, dann kann eine weitere Verschwenkung des Femurs gegenüber dem Hüftknochen nur dadurch erreicht werden, daß um den Anlagepunkt herum verschwenkt wird, und dies führt zwangsläufig dazu, daß die kugelige Gelenkfläche aus der Lagerschale ausgehoben wird, d.h. dabei vergrößert sich der Abstand der Mittelpunkte von Lagerschale einerseits und kugeliger Gelenkfläche andererseits, die bei der normalen Einpassung der kugeligen Gelenkfläche in die Lagerschale zusammenfallen. Wenn man also feststellt, daß diese Mittelpunkte sich voneinander entfernen, daß also eine Luxation im Hüftgelenk eintritt, dann ist dies ein Zeichen dafür, daß ein Anschlag die weitere Schwenkbewegung des Femurs gegenüber dem Hüftgelenk behindert.

Um diese Entfernung der Mittelpunkte bestimmen zu können, ist es günstig, wenn man die Lagedaten sowohl der Lagerschale als auch des Prothesenschaftes mit Hilfe eines Navigationssystems bestimmt. Derartige Navigationssysteme sind bei chirurgischen Operationen üblich, es handelt sich dabei um Systeme, die die Lage von sogenannten Markierelementen im Raum bestimmen können, beispielsweise durch Aussenden und Empfangen von Infrarotstrahlung, die von diesen Markierelementen reflektiert wird. Diese Markierelemente werden an den Teilen starr befestigt, deren Lage zu bestimmen ist, so daß auf diese Weise auch deren Lage im Raum vom Navigationssystem bestimmt wird.

Insbesondere kann vorgesehen sein, daß man Markierelemente des Navigationssystems am Hüftknochen und am Femur plaziert. Durch diese Markierelemente werden die Positionen des Hüftknochens einerseits und des Femurs andererseits überwacht, wobei das Navigationssystem in allen Fällen sowohl die Position als auch die Orientierung im Raum bestimmt, die Gesamtheit dieser Daten wird nachstehend als "Lage" bezeichnet.

Besonders vorteilhaft ist es, wenn man die Lage der Mittelpunkte der Lagerschale und der kugeligen Gelenkfläche durch Verschwenkung des Femurs gegenüber dem Hüftknochen um einen Winkel bestimmt, der unterhalb des maximalen Schwenkwinkels liegt. Bei einer solchen Verschwenkung fallen beide Mittelpunkte zusammen, und die Markierelemente bewegen sich auf einer Kugelschale um diese Mittelpunkte, so daß diese Mittelpunkte zu bestimmen sind. Man erhält also dann durch die Lagebestimmung des Femurs und des Hüftknochens auch die genaue Lage der Mittelpunkte sowohl der Lagerschale als auch der kugeligen Gelenkfläche und kann beim Verschwenken des Hüftknochens über den maximalen Schwenkwinkel hinaus die Luxation des Hüftgelenks beobachten, also die Vergrößerung des Abstandes der Mittelpunkte von Lagerschale einerseits und kugeliger Gelenkfläche andererseits.

Als Prothesenschaft verwendet man vorzugsweise einen Testschaft, also eine Prothese, die nur vorläufig in den Femur eingesetzt wird und nur zu Meß- und Justierzwecken verwendet wird, sobald die genaue Lagebestimmung erfolgt ist, wird dieser Testschaft durch den eigentlichen Prothesenschaft ersetzt, der entsprechende Abmessungen hat und in gleicher Weise im Femur plaziert wird.

Bei einer besonders bevorzugten Ausführungsform ist vorgesehen, daß man auf einer Anzeige eine Lagerschale abbildet und längs deren Umfang die maximalen Schwenkwinkel darstellt. Der Operateur kann dann an dieser Anzeige sofort die maximalen Schwenkwinkel ablesen, die sich beim Verschwenken des Femurs in unterschiedlichen Schwenkrichtungen ergeben.

Insbesondere kann man dabei die Umfangswinkelbereiche der Lagerschale markieren, in denen der maximale Schwenkwinkel unter einem erwünschten Wert liegt. Während die maximalen Schwenkwinkel in bestimmten Richtungen durch anatomische Gegebenheiten bestimmt werden, kann man auf diese Weise erkennen, daß der maximal erreichbare Schwenkwinkel unter Umständen unterhalb dieses anatomischen Maximalwinkels liegt und durch unerwünschte Anschläge hervorgerufen sein muß. Diese Anschläge kann der Operateur gegebenenfalls entfernen, beispielsweise durch Entfernung von Knochenmaterial oder durch eine Änderung der Position der Lagerschale, eventuell auch durch Verwendung einer anderen Lagerschale, z.B. einer asymmetrischen Lagerschale, oder durch Verwendung eines anderen Prothesenschaftes, der aufgrund anderer Dimensionen den unerwünschten Anschlag vermeidet.

Es ist dann günstig, daß man nach der Entfernung der Begrenzung der Schwenkbewegung in einem bestimmten Umfangswinkelbereich in diesem erneut den maximalen Schwenkwinkel bestimmt, um sicher zu sein, daß der unerwünschte Anschlag so weit entfernt worden ist, daß der gewünschte Maximalwinkel erreicht werden kann.

Der Erfindung liegt weiterhin die Aufgabe zugrunde, eine Vorrichtung zur Durchführung dieses Verfahrens zu schaffen.

Eine solche Vorrichtung ist gemäß der Erfindung dadurch gekennzeichnet, daß ein Navigationssystem vorgesehen ist zur Lagebestimmung des Hüftknochens und des Femurs, daß eine Datenverarbeitungseinrichtung vorgesehen ist, welche aus den derart bestimmten Lagedaten des Hüftknochens und des Femurs die Mittelpunkte der Lagerschale und der kugeligen Gelenkfläche und deren gegenseitigen Abstand berechnet und die ein Signal erzeugt, wenn der gegenseitige Abstand der Mittelpunkte einen bestimmten Wert übersteigt.

Insbesondere kann die Datenverarbeitungseinrichtung beim Erzeugen des Signals die Schwenkrichtung des Femurs relativ zum Hüftknochen bestimmen, so daß dem Operateur für jede Schwenkrichtung der maximale Schwenkwinkel angezeigt werden kann.

Bei einer bevorzugten Ausführungsform ist eine Anzeige vorgesehen, auf der die Datenverarbeitungseinrichtung eine Lagerschale abbildet und längs deren Umfang die maximalen Schwenkwinkel darstellt.

Es kann dabei vorgesehen sein, daß die Datenverarbeitungseinrichtung die Umfangswinkelbereiche der Lagerschale markiert, in denen der maximale Schwenkwinkel unter einem erwünschten Wert liegt.

Vorzugsweise ist der Prothesenschaft ein Testschaft.

Die nachfolgende Beschreibung bevorzugter Ausführungsformen der Erfindung dient im Zusammenhang mit der Zeichnung der näheren Erläuterung. Es zeigen:

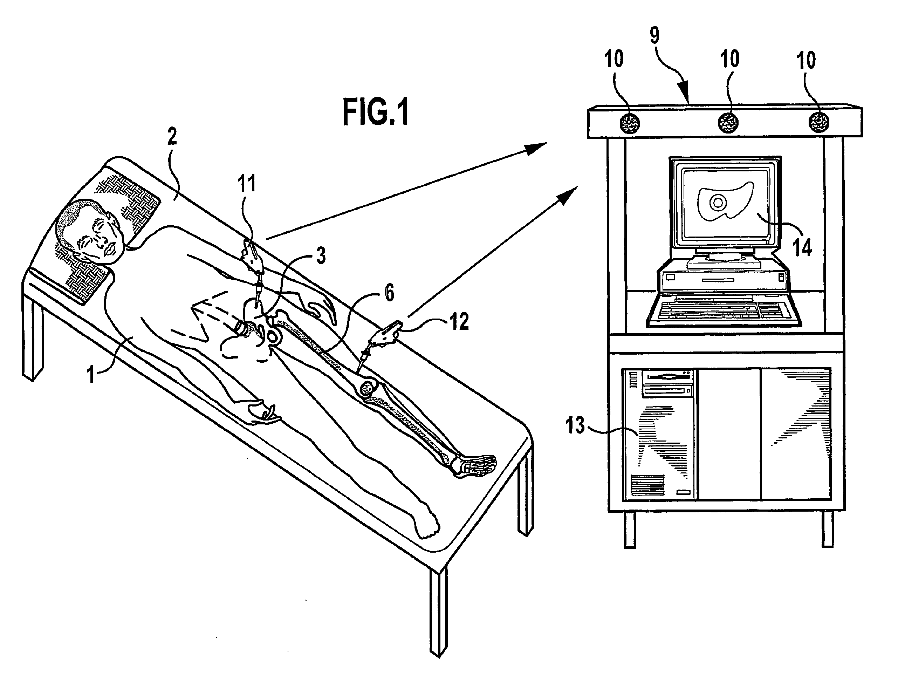

- Figur 1:: eine schematische Ansicht einer Vorrichtung zur Bestimmung des maximalen Schwenkwinkels eines Femurs gegenüber einem Hüftknochen;

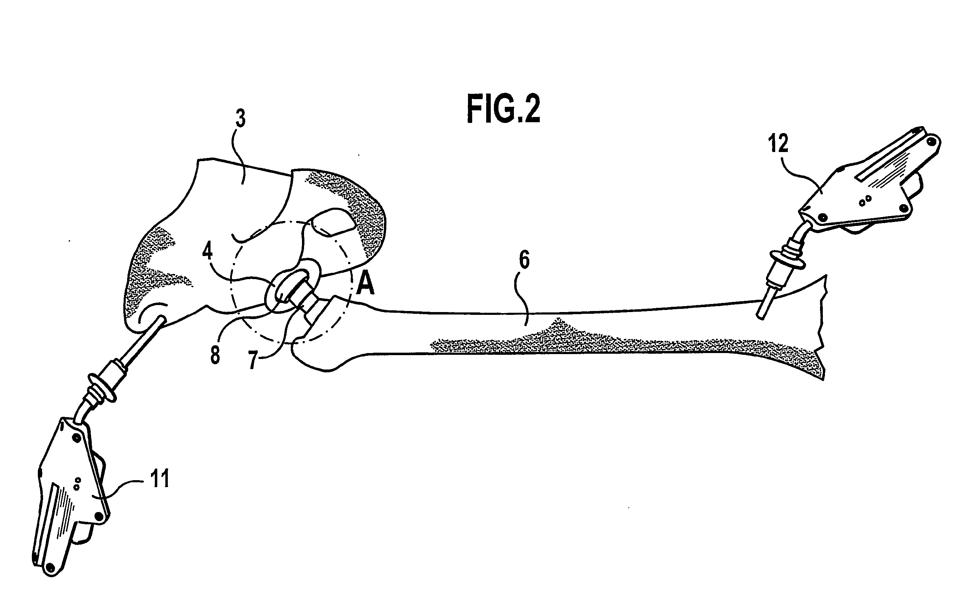

- Figur 2: eine vergrößerte Darstellung des Hüftgelenkes mit eingesetzter Hüftgelenkprothese und mit Markierelementen an Femur und Hüftknochen;
- Figur 3a:: eine Schnittansicht durch das Hüftgelenk im Bereich A in Figur 2 mit normaler Lagerung der kugeligen Gelenkfläche des Prothesenschaftes in der Lagerschale;
- Figur 3b:: eine Ansicht ähnlich Figur 3a bei aus der Lagerschale ausgehobener kugeliger Gelenkfläche des Prothesenschaftes und
- Figur 4:: eine schematische Darstellung einer Anzeige einer Lagerschale mit markiertem Luxationsbereich.

In einem Operationssaal liegt ein Patient 1 auf einem Operationstisch 2, bei dem ein künstliches Hüftgelenk eingesetzt werden soll. Dazu wird in den Hüftknochen 3 eine Lagerschale 4 mit einer im wesentlichen halbkugeligen konkaven Lagerfläche 5 eingesetzt und in den Femur 6 ein Prothesenschaft 7, der an seinem aus dem Femur 6 herausragenden Ende eine konvexe, kugelige Gelenkfläche 8 trägt. Diese kugelige Gelenkfläche 8 hat denselben Durchmesser wie die konkave kugelige Lagerfläche 5 und greift in diese so ein, daß die Mittelpunkte der kugeligen Gelenkfläche 8 und der Lagerfläche 5 zusammenfallen, es bildet sich ein Kugelgelenk aus, welches eine Verschwenkung des Femurs 6 gegenüber dem Hüftknochen 3 in allen Richtungen ermöglicht.

Um diese Verschwenkbewegung verfolgen zu können, ist ein Navigationssystem 9 vorgesehen, welches die Lage, also die Position und die Orientierung, von Hüftknochen 3 und Femur 6 im Raum laufend bestimmt. Das Navigationssystem 9 umfaßt mehrere im Abstand zueinander angeordnete Sende- und Empfangseinrichtungen 10 sowie zwei Markierelemente 11, 12, die beispielsweise über Knochenschrauben starr mit dem Hüftknochen 3 bzw. dem Femur 6 verbunden sind. Die Markierelemente 11, 12 empfangen an verschiedenen im Abstand zueinander angeordneten Stellen von den Sende- und Empfangseinrichtungen 10 ausgesandte Strahlung, beispielsweise Infrarotstrahlung, und reflektieren diese auf die Sende- und Empfangseinrichtungen 10. Das Navigationssystem 9 kann aufgrund dieser reflektierten Strahlung die Lage der Markierelemente 11, 12 im Raum feststellen und damit die Lage des Hüftknochens 3 und des Femurs 6.

Dem Navigationssystem 9 ist eine Datenverarbeitungseinrichtung 13 zugeordnet, welche die vom Navigationssystem 9 ermittelten Lagedaten empfängt und weiterverarbeitet, außerdem ein Bildschirm 14, auf dem die Datenverarbeitungseinrichtung 13 diese Daten oder daraus errechnete Daten darstellt.

Der Operateur wird die nachfolgenden Schritte vorzugsweise unter Verwendung sogenannter Testimplantate durchführen, also unter Verwendung eines Prothesenschaftes 7, der noch nicht der endgültige Prothesenschaft ist, sondern der nur vorläufig testweise in den Femur eingesetzt ist, um mit Hilfe dieses Testschaftes die Kinematik des Gelenkes und die richtige Implantationsposition zu überprüfen. Dasselbe kann hinsichtlich der Lagerschale 4 gelten, auch hier ist es möglich, eine Testschale einzusetzen. Diese Testimplantate werden nach erfolgreicher Ausrichtung und Positionierung entfernt und durch endgültige Implantate ersetzt, die die gleiche Geometrie aufweisen und in gleicher Weise implantiert werden wie die Testimplantate.

Zur Bestimmung der Beweglichkeit des Femurs gegenüber dem Hüftknochen werden zunächst die Lagen der Mittelpunkte der Lagerschale 4 einerseits und der kugeligen Gelenkfläche 8 andererseits relativ zum Hüftknochen 3 bzw. zum Femur 6 bestimmt. Dazu wird der Femur 6 relativ zum Hüftknochen 3 in verschiedenen Richtungen verschwenkt, da das Hüftgelenk ein Kugelgelenk ist, bewegen sich dabei die Markierelemente 11 und 12 auf Kugelflächen, deren Mittelpunkt durch den Mittelpunkt des Kugelgelenkes definiert wird. Bei normaler Lagerung ziehen die Muskeln die kugelige Gelenkfläche in die Lagerschale 4 hinein, so daß die kugelige Gelenkfläche 8 flächig an der Lagerfläche 5 anliegt, die Mittelpunkte der Lagerschale 4 und der kugeligen Gelenkfläche 8 fallen daher zusammen. Durch die beschriebene Verschwenkbewegung des Femurs läßt sich also die Lage beider Mittelpunkte in gleicher Weise relativ zum Hüftknochen 3 einerseits und zum Femur 6 andererseits bestimmen.

In einem zweiten Schritt prüft der Operateur nunmehr, wie weit der Femur in dem Hüftgelenk in verschiedenen Richtungen verschwenkt werden kann. Dazu wird der Femur gegenüber dem Hüftknochen so weit wie möglich verschwenkt.

In einigen Richtungen wird diese Verschwenkbewegung durch das umgebende Muskelgewebe begrenzt, darüber hinausgehende Verschwenkungen werden dadurch verhindert.

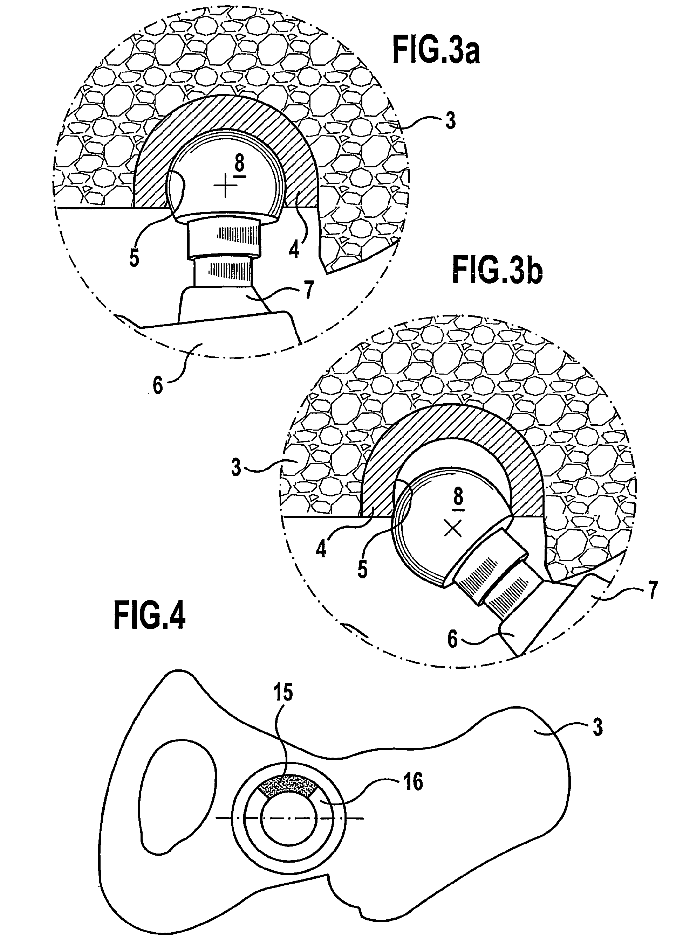

In anderen Richtungen jedoch, in denen das Muskelgewebe eine weitere Verschwenkbewegung ermöglichen würde, kann diese Verschwenkbewegung dadurch behindert werden, daß die beiden gegeneinander bewegten Teile aneinander anschlagen. Dies kann ein Anschlag zwischen knöchernen Strukturen des Femurs und des Hüftknochens sein, es kann auch die knöcherne Struktur des einen Teils am Prothesenteil des anderen Teils anschlagen, es können auch beide Prothesenteile aneinander anschlagen, also Lagerschale und Prothesenschaft. Wenn das der Fall ist und wenn der Operateur dann die Schwenkbewegung des Femurs fortsetzt, bildet der Anschlagpunkt ein Widerlager, und dies führt dazu, daß die kugelige Gelenkfläche 8 aus der Lagerschale 4 ausgehoben wird, es erfolgt also eine Luxation des Hüftgelenkes, wie dies in Figur 3b dargestellt ist. Dabei entfernen sich die Mittelpunkte der Lagerfläche 5 einerseits und der kugeligen Gelenkfläche 8 andererseits voneinander, die vor dem Anschlagen der beiden bewegten Teile dieselbe Lage eingenommen haben, also praktisch einen Abstand Null.

Die Datenverarbeitungseinrichtung erhält über das Navigationssystem 9 laufend Lagedaten sowohl des Hüftknochens 3 als auch des Femurs 6 und kann aus diesen Lagedaten die jeweiligen Lagen der Mittelpunkte der Lagerfläche 5 und der kugeligen Gelenkfläche 8 berechnen und damit auch deren Abstand.

Sobald ein solcher Abstand auftritt, erzeugt die Datenverarbeitungseinrichtung ein Signal, aus dem der Operateur entnehmen kann, daß eine Luxation beginnt, daß also ein Anschlag erfolgt sein muß. Die Datenverarbeitungseinrichtung kann aus den Lagedaten außerdem feststellen, in welche Richtung der Femur dabei gegenüber dem Hüftknochen verschwenkt worden ist, der Operateur erhält also zusätzlich auch eine Information darüber, bei welchem Umfangswinkel der Lagerschale 4 dieser Anschlag aufgetreten ist.

Zusätzlich kann dem Operateur auf dem Bildschirm eine Abbildung der Lagerschale 4 zur Verfügung gestellt werden, beispielsweise in Form eines Ringes, und in dieser Abbildung kann der Umfangswinkelbereich der Lagerschale markiert werden, in dem ein Anschlag erfolgt ist, in dem also eine Luxation aufgetreten ist. Eine solche Abbildung ist schematisch in Figur 4 dargestellt, dort ist ein Bereich 15 im ringförmigen Bild 16 einer Lagerschale markiert, und in diesem Bereich ergibt sich eine Luxation, wenn der Operateur den Femur in diesem Umfangsbereich der Lagerschale verschwenkt. Die Datenverarbeitungseinrichtung kann aus den Lagedaten auch die Schwenkwinkel berechnen, so daß der Operateur gegebenenfalls zusätzlich eine Information darüber erhalten kann, ob die Luxation erst eintritt, wenn ein maximal erwünschter Schwenkwinkel überschritten wird, dann ist der Anschlag unschädlich, weil er erst bei Schwenkwinkeln auftritt, die normalerweise nicht benötigt werden, oder ob die Luxation schon bei einem Schwenkwinkel auftritt, der unter dem maximal gewünschten Schwenkwinkel liegt, dann ist eine solche Begrenzung der Schwenkbewegung störend und muß beseitigt werden.

Dafür stehen dem Operateur mehrere Möglichkeiten zur Verfügung, beispielsweise kann er im Anschlagsbereich knöcherne Strukturen entfernen, es ist auch möglich, andere Prothesenteile zu verwenden, die in diesem Bereich eine geringere Baugröße haben und daher einen Anschlag vermeiden, es besteht die Möglichkeit, asymmetrische Lagerschalen zu verwenden, die in bestimmten Winkelbereichen zurückgesetzte Geometrien aufweisen. Nach einer solchen Entfernung eines Anschlages wird das beschriebene Verfahren wiederholt, um festzustellen, ob nach Entfernung des Anschlages der maximal erwünschte Schwenkwinkel des Femurs ohne Luxation erreicht werden kann.

Die Feststellung einer Luxation, d.h. einer Entfernung der Mittelpunkte von Lagerfläche 5 und kugeliger Gelenkfläche 8 ist also immer ein Zeichen dafür, daß die Verschwenkbewegung des Femurs in einer bestimmten Richtung durch einen Anschlag begrenzt wird, und diese Information kann der Operateur zur Überprüfung der erwünschten Beweglichkeit des Hüftgelenkes in der beschriebenen Weise verwenden.

## Patentansprüche

1. Verfahren zur Bestimmung der Beweglichkeit einer Hüftgelenkprothese mit einer in den Hüftknochen einsetzbaren Lagerschale und einem in den Femur einsetzbaren Prothesenschaft, der mit einer kugeligen Gelenkfläche in der Lagerschale verschwenkbar gelagert ist, **dadurch gekennzeichnet, daß** man den Femur mit dem Prothesenschaft in verschiedenen Richtungen so weit wie möglich verschwenkt, daß man feststellt, ob sich dabei der Mittelpunkt der Lagerschale und der Mittelpunkt der kugeligen Gelenkfläche voneinander entfernen, und daß man **dadurch** für jede Schwenkrichtung den maximalen Schwenkwinkel bestimmt, bei dem die Mittelpunkte beginnen, sich voneinander zu entfernen.

2. Verfahren nach Anspruch 1, **dadurch gekennzeichnet, daß** man die Lagedaten sowohl der Lagerschale als auch des Prothesenschaftes mit Hilfe eines Navigationssystems bestimmt.

3. Verfahren nach Anspruch 2, **dadurch gekennzeichnet, daß** man Markierelemente des Navigationssystems am Hüftgelenkknochen und am Femur plaziert.

4. Verfahren nach einem der Ansprüche 2 oder 3, **dadurch gekennzeichnet, daß** man die Lage der Mittelpunkte der Lagerschale und der kugeligen Gelenkfläche durch Verschwenkung des Femurs gegenüber dem Hüftknochen um einen Winkel bestimmt, der unterhalb des maximalen Schwenkwinkels liegt.

5. Verfahren nach einem der voranstehenden Ansprüche, **dadurch gekennzeichnet, daß** man als Prothesenschaft einen Testschaft verwendet.

6. Verfahren nach einem der voranstehenden Ansprüche, **dadurch gekennzeichnet, daß** man auf einer Anzeige eine Lagerschale abbildet und längs deren Umfang die maximalen Schwenkwinkel darstellt.

7. Verfahren nach Anspruch 6, **dadurch gekennzeichnet, daß** man Umfangswinkelbereiche der Lagerschale markiert, in denen der maximale Schwenkwinkel unter einem erwünschten Wert liegt.

8. Verfahren nach einem der voranstehenden Ansprüche, **dadurch gekennzeichnet, daß** man die Begrenzung der Schwenkbewegung in einem bestimmten Umfangswinkelbereich entfernt und in diesem Umfangswinkelbereich erneut den maximalen Schwenkwinkel bestimmt.

9. Vorrichtung zur Bestimmung der Beweglichkeit einer Hüftgelenkprothese mit einer in den Hüftknochen (3) einsetzbaren Lagerschale (4) und einem in den Femur (6) einsetzbaren Prothesenschaft (7), der mit einer kugeligen Gelenkfläche (8) in der Lagerschale (4) verschwenkbar gelagert ist, wobei ein Navigationssystem (9) vorlagert Navigationssystem (9) vorgesehen ist zur Lagebestimmung des Hüftknochens (3) und des Femurs (6), und eine Datenverarbeitungseinrichtung (13) vorgesehen ist, **dadurch gekennzeichnet, daß** die Datenverarbeitungseinrichtung (13) aus den bestimmten Lagedaten des Hüftknochens (3) und des Femurs (6) die Mittelpunkte der Lagerschale (4) und der kugeligen Gelenkfläche (8) und deren gegenseitigen Abstand berechnet und die ein Signal erzeugt, wenn der gegenseitige Abstand der Mittelpunkte einen bestimmten Wert übersteigt.

10. Vorrichtung nach Anspruch 9, **dadurch gekennzeichnet, daß** die Datenverarbeitungseinrichtung (13) beim Erzeugen des Signals die Schwenkrichtung des Femurs (6) relativ zum Hüftknochen (3) bestimmt.

11. Vorrichtung nach Anspruch 10, **dadurch gekennzeichnet, daß** eine Anzeige (14) vorgesehen ist, auf der die Datenverarbeitungseinrichtung (13) eine Lagerschale (16) abbildet und längs deren Umfang die maximalen Schwenkwinkel darstellt.

12. Vorrichtung nach Anspruch 11, **dadurch gekennzeichnet, daß** die Datenverarbeitungseinrichtung (13) die Umfangswinkelbereiche der Lagerschale (16) markiert, in denen der maximale Schwenkwinkel unter einem erwünschten Wert liegt.

13. Vorrichtung nach einem der Ansprüche 9 bis 12, **dadurch gekennzeichnet, daß** der Prothesenschaft (7) ein Testschaft ist.

## Claims

1. Method for determination of the mobility of a hip joint prosthesis comprising a bearing cup that can be inserted in the hip bone and a prosthetic shaft that can be inserted in the femur and is pivotally mounted by a spherical joint surface in the bearing cup, **characterised in that** the femur with the prosthetic shaft is pivoted as far as possible in various directions, **in that** it is determined whether the central point of the bearing cup and the central point of the spherical joint surface move apart from one another during this process, and **in that**, for each pivoting direction, the maximum pivoting angle is thus determined, at which the central points begin to move apart from one another.

2. Method according to claim 1, **characterised in that** the positional data both of the bearing cup and also of the prosthetic shaft is determined with the aid of a navigation system.

3. Method according to claim 2, **characterised in that** marking elements of the navigation system are placed on the hip joint bone and on the femur.

4. Method according to either of claims 2 or 3, **characterised in that** the position of the central points of the bearing cup and the spherical joint surface is determined by pivoting the femur with respect to the hip bone by an angle which is below the maximum pivoting angle.

5. Method according to any one of the preceding claims, **characterised in that** a test shaft is used as the prosthetic shaft.

6. Method according to any one of the preceding claims, **characterised in that** a bearing cup is reproduced on a display and the maximum pivoting angle is shown along the periphery thereof.

7. Method according to claim 6, **characterised in that** there are marked peripheral angle ranges of the bearing cup, in which the maximum pivoting angle is below a desired value.

8. Method according to any one of the preceding claims, **characterised in that** the limitation of the pivoting movement in a specific peripheral angle range is removed and the maximum pivoting angle is determined afresh in this peripheral angle range.

9. Device for determination of the mobility of a hip joint prosthesis comprising a bearing cup (4) that can be inserted in the hip bone (3) and a prosthetic shaft (7) that can be inserted in the femur (6) and is pivotally mounted by a spherical joint surface (8) in the bearing cup (4), wherein a navigation system (9) is provided for determining the position of the hip bone (3) and the femur (6), and a data processing device (13) is provided, **characterised in that** the data processing device (13) calculates the central points of the bearing cup (4) and the spherical joint surface (8) and their mutual spacing from the determined positional data of the hip bone (3) and the femur (6) and which generates a signal when the mutual spacing of the central points exceeds a specific value.

10. Device according to claim 9, **characterised in that** the data processing device (13), on generation of the signal, determines the pivoting direction of the femur (6) relative to the hip bone (3).

11. Device according to claim 10, **characterised in that** a display (14) is provided on which the data processing device (13) reproduces a bearing cup (16) and shows the maximum pivoting angle along the periphery thereof.

12. Device according to claim 11, **characterised in that** the data processing device (13) marks the peripheral angle ranges of the bearing cup (16), in which the maximum pivoting angle is below a desired value.

13. Device according to any one of claims 9 to 12, **characterised in that** the prosthetic shaft (7) is a test shaft.

## Revendications

1. Procédé pour déterminer la mobilité d'une prothèse de la hanche qui comprend une coque de palier à intégrer dans l'os de la hanche et une tige de prothèse à intégrer dans le fémur, laquelle est montée avec possibilité de pivotement dans la coque de palier via une surface d'articulation sphérique, **caractérisé en ce que** le fémur est pivoté avec la tige de prothèse dans différentes directions aussi loin que possible, **en ce que** l'on constate si le centre de la coque de palier et le centre de la surface d'articulation sphérique s'éloignent alors l'un de l'autre, et **en ce que** l'on détermine ainsi pour chaque direction de pivotement l'angle de pivotement maximum pour lequel les centres commencent à s'éloigner l'un de l'autre.

2. Procédé selon la revendication 1, **caractérisé en ce que** les données de position sont déterminées à l'aide d'un système de navigation aussi bien pour la coque de palier que pour la tige de prothèse.

3. Procédé selon la revendication 2, **caractérisé en ce que** l'on place des éléments de marquage du système de navigation sur l'os de la hanche et sur le fémur.

4. Procédé selon l'une des revendications 2 ou 3, **caractérisé en ce que** l'on détermine la position du centre de la coque de palier et du centre de la surface articulaire sphérique par pivotement du fémur par rapport à la hanche sur un angle qui est inférieur à l'angle de pivotement maximum.

5. Procédé selon l'une des revendications précédentes, **caractérisé en ce que** l'on utilise une tige de test à titre de tige de prothèse.

6. Procédé selon l'une des revendications précédentes, **caractérisé en ce que** l'on forme l'image d'une coque de palier sur un affichage et que l'on présente l'angle de pivotement maximum le long de sa périphérie.

7. Procédé selon la revendication 6, **caractérisé en ce que** l'on marque des plages angulaires périphériques de la coque de palier dans lesquelles l'angle de pivotement maximum est inférieur à une valeur souhaitée.

8. Procédé selon l'une des revendications précédentes, **caractérisé en ce que** l'on supprime la limitation du mouvement de pivotement dans une plage angulaire périphérique déterminée et **en ce que** l'on détermine à nouveau l'angle de pivotement maximum dans cette plage angulaire périphérique.

9. Dispositif pour déterminer la mobilité d'une prothèse de la hanche qui comprend une coque de palier (4) à intégrer dans l'os de la hanche (3) et une tige de prothèse (7) à intégrer dans le fémur (6), laquelle est montée avec possibilité de pivotement dans la coque de palier (4) via une surface d'articulation sphérique (8), dans lequel il est prévu un système de navigation (9) pour déterminer la position de l'os de la hanche (3) et du fémur (6), et il est prévu un système de traitement de données (13), **caractérisé en ce que** le système de traitement de données (13) calcule, à partir des données de position déterminée de l'os de la hanche (3) et du fémur (6), le centre de la coque de palier (4) et le centre de la surface d'articulation sphérique (8), ainsi que leur distance mutuelle, et génère un signal lorsque la distance mutuelle des centres dépasse une valeur déterminée.

10. Dispositif selon la revendication 9, **caractérisé en ce que** le système de traitement de données (13) détermine, lors de la génération du signal, la direction de pivotement du fémur (6) par rapport à l'os de la hanche (3).

11. Dispositif selon la revendication 10, **caractérisé en ce qu'**il est prévu un affichage (14) sur lequel le système de traitement de données (13) forme l'image d'une coque de palier (16) et illustre l'angle de pivotement maximum le long de sa périphérie.

12. Dispositif selon la revendication 11, **caractérisé en ce que** le système de traitement de données (13) marque les plages angulaires périphériques de la coque de palier (16) dans lesquelles l'angle de pivotement maximum tombe au-dessous d'une valeur souhaitée.

13. Dispositif selon l'une des revendications 9 à 12, **caractérisé en ce que** la tige de prothèse (7) est une tige de test.
